# EUROPEAN PATENT SPECIFICATION

(11) **EP 3 149 203 B1**
(45) Date of publication and mention of the grant of the patent: **04.12.2019**
(21) Application number: 15766242.0
(22) Date of filing: 27.05.2015
(51) Int. Cl.: C12Q 1/68

(54) **IN VITRO METHOD FOR THE DETECTION AND QUANTIFICATION OF HIV-2**
IN-VITRO-VERFAHREN ZUR ERKENNUNG UND QUANTIFIZIERUNG VON HIV-2
PROCÉDÉ IN VITRO POUR LA DÉTECTION ET LA QUANTIFICATION DU VIH-2

(30) Priority: 27.05.2014 EP 14169958
(43) Date of publication of application: 05.04.2017
(73) Proprietor: Université Paris Descartes, 75006 Paris (FR); Université Paris Diderot, 75013 Paris (FR); Institut National de la Santé et de la Recherche Médicale (INSERM), 75013 Paris (FR); Assistance Publique-Hôpitaux de Paris, 75004 Paris (FR); Université de Rouen, 76821 Mont-Saint-Aignan (FR); CHU de Rouen, 76031 Rouen (FR)
(72) Inventor: ROUZIOUX, Christine, F-75015 Paris (FR); PLANTIER, Jean-Christophe, F-76230 Bois-Guillaume (FR); AVETTAND-FENOEL, Véronique, F-75015 Paris (FR); DAMOND, Florence, F-94160 Saint Mande (FR); GUEUDIN, Marie, F-76000 Rouen (FR); DESCAMPS, Diane, F-75005 Paris (FR)
(74) Representative: Vial, Lionel
(86) International application number: PCT/IB2015/001191
(87) International publication number: WO 2015/181627

(56) References cited:
- EP-A2- 0 887 427
- WO-A1-2012/168480
- FR-A1- 2 882 063
- US-B1- 6 303 293
- CHARLOTTE CHARPENTIER ET AL: "Genotypic resistance profiles of HIV-2-treated patients in Côte d'Ivoire, West Africa", AIDS, 1 February 2014 (2014-02-01), page 1, XP055139003, ISSN: 0269-9370, DOI: 10.1097/QAD.0000000000000244
- F. DAMOND ET AL: "Plasma RNA Viral Load in Human Immunodeficiency Virus Type 2 Subtype A and Subtype B Infections", JOURNAL OF CLINICAL MICROBIOLOGY, vol. 40, no. 10, 1 October 2002 (2002-10-01), pages 3654-3659, XP055139841, ISSN: 0095-1137, DOI: 10.1128/JCM.40.10.3654-3659.2002
- F. DAMOND ET AL: "Improved Sensitivity of Human Immunodeficiency Virus Type 2 Subtype B Plasma Viral Load Assay", JOURNAL OF CLINICAL MICROBIOLOGY, vol. 43, no. 8, 1 August 2005 (2005-08-01) , pages 4234-4236, XP055139929, ISSN: 0095-1137, DOI: 10.1128/JCM.43.8.4234-4236.2005 cited in the application
- SCHUTTEN MARTIN ET AL: "Development of a real-time quantitative RT-PCR for the detection of HIV-2 RNA in plasma", JOURNAL OF VIROLOGICAL METHODS, ELSEVIER BV, NL, vol. 88, no. 1, 1 July 2000 (2000-07-01), pages 81-87, XP002349518, ISSN: 0166-0934, DOI: 10.1016/S0166-0934(00)00177-4
- DATABASE EMBL [Online] 19 December 2001 (2001-12-19), "Sequence 13 from patent US 6303293.", XP002729720, retrieved from EBI accession no. EM_PAT:AR172157 Database accession no. AR172157
- DATABASE Geneseq [Online] 11 March 1999 (1999-03-11), "HIV-2 long terminal repeat (LTR) region probe.", XP002729721, retrieved from EBI accession no. GSN:AAV63678 Database accession no. AAV63678
- DATABASE Geneseq [Online] 31 January 2013 (2013-01-31), "HIV-2 DNA amplifying real-time-PCR primer hiv2-f2, SEQ ID:51.", XP002729722, retrieved from EBI accession no. GSN:BAJ09419 Database accession no. BAJ09419
- SÉVERINE DELARUE ET AL: "Highly sensitive plasma RNA quantification by real-time PCR in HIV-2 group A and group B infection", JOURNAL OF CLINICAL VIROLOGY, vol. 58, no. 2, 1 October 2013 (2013-10-01), pages 461-467, XP055139001, ISSN: 1386-6532, DOI: 10.1016/j.jcv.2013.08.003 cited in the application
- J.-C. PLANTIER ET AL: "Rapid Discrimination between Human Immunodeficiency Virus Type 2 Groups A and B by Real-Time PCR", JOURNAL OF CLINICAL MICROBIOLOGY, vol. 42, no. 12, 1 December 2004 (2004-12-01), pages 5866-5870, XP055139940, ISSN: 0095-1137, DOI: 10.1128/JCM.42.12.5866-5870.2004
- LINDA M. STYER ET AL: "Validation and clinical use of a sensitive HIV-2 viral load assay that uses a whole virus internal control", JOURNAL OF CLINICAL VIROLOGY, vol. 58, 1 December 2013 (2013-12-01), pages e127-e133, XP055139004, ISSN: 1386-6532, DOI: 10.1016/j.jcv.2013.09.007 cited in the application
- MING CHANG ET AL: "Validation for clinical use of a novel HIV-2 plasma RNA viral load assay using the Abbott m2000 platform", JOURNAL OF CLINICAL VIROLOGY, vol. 55, no. 2, 1 October 2012 (2012-10-01), pages 128-133, XP055139007, ISSN: 1386-6532, DOI: 10.1016/j.jcv.2012.06.024 cited in the application
- AVETTAND-FENOEL VÉRONIQUE ET AL: "New sensitive one-step real-time duplex PCR method for group A and B HIV-2 RNA load", JOURNAL OF CLINICAL MICROBIOLOGY,, vol. 52, no. 8, 1 August 2014 (2014-08-01) , pages 3017-3022, XP009180050, ISSN: 1098-660X
- ROUET FRANÇOIS ET AL: "Field evaluation of a rapid human immunodeficiency virus (HIV) serial serologic testing algorithm for diagnosis and differentiation of HIV type 1 (HIV-1), HIV-2, and dual HIV-1-HIV-2 infections in West African pregnant women.", JOURNAL OF CLINICAL MICROBIOLOGY SEP 2004, vol. 42, no. 9, September 2004 (2004-09), pages 4147-4153, ISSN: 0095-1137
- DAMOND F ET AL: "Evaluation of an upgraded version of the roche cobas ampliprep/cobas TaqMan HIV-1 Test for HIV-1 load quantification", JOURNAL OF CLINICAL MICROBIOLOGY 20100401 AMERICAN SOCIETY FOR MICROBIOLOGY USA, vol. 48, no. 4, 1 April 2010 (2010-04-01), pages 1413-1416, ISSN: 0095-1137

## Description

### Field of the invention

The present invention relates to an *in vitro* method for the detection and quantification of Human Immunodeficiency Virus (HIV) 2.

### Technical background

HIV-2 is characterized by less efficient transmission through the sexual and vertical routes than HIV-1, and by a slower natural clinical course. Nevertheless, HIV-2 infection eventually leads to AIDS. HIV-2 infection must be distinguished from HIV-1 infection, as HIV-2 is naturally resistant to non-nucleoside reverse transcriptase inhibitors, T20, and some protease inhibitors, and as patient follow-up differs from that of HIV-1 infection.

Compared to HIV-1, HIV-2 is characterized by lower viral replication. In the French ANRS cohort CO5 of HIV-2-infected patients (1009 patients in January 2014), 61% of untreated patients have plasma viral loads below 250 copies/mL (cp/mL). Likewise, in a British study, only 8% of patients with CD4 >500 cells/mm3 and 62% of patients with CD4 <300 cells/mm3 had detectable viral load, implying that 38% of patients had undetectable viral load in an assay with a quantification limit of 100 copies/ml.

Clinical management of HIV-2 infection is hampered by the lack of validated commercial RNA viral load assays. In-house assays are therefore widely used, such as the assay described by Damond et al. (2005) J. Clin. Microbiol. 43:4234-6 which is used for quantifying the viral load in the French cohort CO5 of HIV-2-infected patients.

However, the ACHIEV2E international collaboration on HIV-2 infection showed that plasma HIV-2 RNA values vary considerably between laboratories. Besides, the high genetic diversity of HIV-2, with 9 groups designated A to I, of which only groups A and B are epidemic, also represents an obstacle to accurate viral load quantification: previous studies have thus shown that group B viruses are particularly difficult to quantify with the current assays. In addition, the current HIV-2 assays also suffer from low sensitivity and accuracy.

It is therefore an object of the invention to overcome these limitations.

### Summary of the invention

The present invention arises from the unexpected identification, by the present inventors, of a combination of two specific target sequences in HIV-2 RNA which duplex amplification by real-time RT-PCR enables efficient detection of group B viruses and provide for a detection limit below 40 copies/mL as well as a quantification limit below 100 copies/ml.

The present invention thus relates to a method for detecting or quantifying Human Immunodeficiency Virus-2 (HIV-2) nucleic acids in a biological sample, comprising:
a) performing a real-time polymerase chain reaction (PCR) or a real-time reverse transcriptase polymerase chain reaction (RT-PCR) on nucleic acids of the biological sample with:
   (i) at least 4 primers respectively comprising or consisting of:
      - sequence SEQ ID NO: 1 or a sequence having at least 90% identity to SEQ ID NO: 1, and
      - sequence SEQ ID NO: 2 or a sequence having at least 90% identity to SEQ ID NO: 2, and
      - sequence SEQ ID NO: 4 or a sequence having at least 90% identity to SEQ ID NO: 4, and
      - sequence SEQ ID NO: 5 or a sequence having at least 90% identity to SEQ ID NO: 5,
      wherein the primers comprise not more than 30 nucleotides, and
   (ii) at least 2 labelled probes respectively comprising or consisting of:
      - sequence SEQ ID NO: 3, a sequence complementary to SEQ ID NO: 3, or a sequence having at least 90% identity to SEQ ID NO: 3 or the complementary thereof, and
      - sequence SEQ ID NO: 6, a sequence complementary to SEQ ID NO: 6, or a sequence having at least 90% identity to SEQ ID NO: 6 or the complementary thereof,
      wherein the probes comprise not more than 30 nucleotides, and
b) determining therefrom the presence or absence and/or the quantity of HIV-2 nucleic acids in the biological sample.

In an embodiment of the invention, the above-defined method for detecting or quantifying Human Immunodeficiency Virus-2 (HIV-2) nucleic acids further comprises determining or quantifying HIV-1 nucleic acids in a biological sample.

The present invention also relates to a kit or a mix for detecting or quantifying HIV-2 nucleic acids, comprising:
a) at least 4 primers respectively comprising or consisting of:
   - sequence SEQ ID NO: 1 or a sequence having at least 90% identity to SEQ ID NO: 1, and
   - sequence SEQ ID NO: 2 or a sequence having at least 90% identity to SEQ ID NO: 2, and
   - sequence SEQ ID NO: 4 or a sequence having at least 90% identity to SEQ ID NO: 4, and
   - sequence SEQ ID NO: 5 or a sequence having at least 90% identity to SEQ ID NO: 5,
   wherein the primers comprise not more than 30 nucleotides, and
b) at least 2 labelled probes respectively comprising or consisting of:
   - sequence SEQ ID NO: 3, a sequence complementary to SEQ ID NO: 3, or a sequence having at least 90% identity to SEQ ID NO: 3 or the complementary thereof, and
   - sequence SEQ ID NO: 6, a sequence complementary to SEQ ID NO: 6, or a sequence having at least 90% identity to SEQ ID NO: 6 or the complementary thereof,
   wherein the probes comprise not more than 30 nucleotides, and
c) optionally additional reagents for performing PCR or RT-PCR.

In an embodiment of the invention, the above-defined kit or mix further comprises primers and labelled probes for detecting or quantifying HIV-1 nucleic acids.

In another embodiment of the invention, the above-defined method for detecting or quantifying Human Immunodeficiency Virus-2 (HIV-2) nucleic acids in a biological sample, kit and mix further comprise at least one primer comprising or consisting of sequence SEQ ID NO: 7 or a sequence having at least 90% identity to SEQ ID NO: 7.

The present invention also relates to the use of the kit or the mix as defined above, for detecting or quantifying HIV-2 nucleic acids of a biological sample.

The present invention also relates to a method, in particular an *in vitro* method for diagnosing HIV-2 infection, or determining HIV-2 viral load, in an individual, comprising the steps of:
(a) performing the method for detecting or quantifying Human Immunodeficiency Virus-2 (HIV-2) nucleic acids in a biological sample taken from the individual as defined above;
(b) determining therefrom whether the individual is infected by HIV-2 or the HIV-2 viral load of the individual.

The present invention also relates to a method, in particular an *in vitro* method, for determining whether an individual is liable to benefit from a treatment with antiretroviral therapy (ART) or from an adjustment of ART, comprising performing the method for diagnosing HIV-2 infection, or determining HIV-2 viral load, as defined above.

The present invention also relates to nucleotide reverse transcriptase inhibitors (NRTIs), Protease inhibitors (Pis) and/or Integrase inhibitors for use in the prevention or treatment of HIV-2 infection in an individual, wherein the individual is determined to be liable to benefit from a treatment with ART or from an adjustment of ART by performing the methods of the invention.

The present invention also relates to a probe, in particular a labelled probe, comprising or consisting of sequence SEQ ID NO: 3, a sequence complementary to SEQ ID NO: 3. The present invention also relates to a kit or a mix as disclosed in the appended claims. The present application also relates to a kit or a mix for detecting or quantifying HIV-2 nucleic acids, comprising:
a) at least 2 primers respectively comprising or consisting of:
   - sequence SEQ ID NO: 1 or a sequence having at least 90% identity to SEQ ID NO: 1, and
   - sequence SEQ ID NO: 2 or a sequence having at least 90% identity to SEQ ID NO: 2, and
b) at least one labelled probes comprising or consisting of:
   - sequence SEQ ID NO: 3, a sequence complementary to SEQ ID NO: 3, or a sequence having at least 90% identity to SEQ ID NO: 3 or the complementary thereof, and
c) optionally additional reagents for performing PCR or RT-PCR.

The present invention further relates to the use of the above-defined probe, kit or mix as defined in the appended claims, for detecting or quantifying HIV-2 nucleic acids of a biological sample.

### Detailed description of the invention

### HIV-2 nucleic acids

Human Immunodeficiency Virus 2 (HIV-2) which infection may lead to Acquired Immune Deficiency Syndrome (AIDS) is well known to one of skill in the art. HIV-2 is a member of the genus *Lentivirus,* part of the *Retroviridae* family. Its replication cycle involves entry of HIV-2 single-stranded RNA into a cell, reverse transcription to yield a single-stranded DNA and then a double-stranded DNA which is integrated in the host cell genome. Accordingly, as intended herein, HIV-2 nucleic acids relate to nucleic acids of any type which harbour the HIV-2 genome in totality or in part. HIV-2 nucleic acids to be detected or quantified according to the invention may thus be RNA, in particular single stranded RNA, or DNA, in particular single stranded or double stranded DNA.

Preferably, the above-defined method, kit or mix, or use for detecting or quantifying Human Immunodeficiency Virus-2 (HIV-2) nucleic acids is for detecting or quantifying HIV-2 RNA.

### Real-time PCR or RT-PCR

Real-time Polymerase Chain Reaction (PCR) and Reverse Transcriptase Polymerase Chain Reaction (RT-PCR) are well-known to one of skill in the art and are also known as quantitative PCR (qPCR) and quantitative RT-PCR (RT-qPCR).

According to the invention, real-time PCR will be conducted for detecting or quantifying HIV-2 DNA while RT-PCR will be conducted for detecting or quantifying HIV-2 RNA.

One of skill in the in art can readily determine from a real-time PCR or RT-PCR whether HIV-2 nucleic acids are present, *i.e*. detect them, and/or quantify HIV-2 nucleic acids. Typically, in real-time PCR or RT-PCR, a signal, generally a fluorescent signal, which intensity is a consequence of the accumulation of amplified DNA is measured by the thermal cycler which runs the PCR or RT-PCR. If, in the course of the PCR or RT-PCR, the intensity of the measured signal, in particular the fluorescent signal, is higher than a signal threshold, generally background signal intensity, it is deduced that amplification has occurred, *i.e*. that HIV-2 nucleic acids are present in the biological sample. Conversely, if no signal intensity higher than background signal intensity is measured in the course of PCR or RT-PCR, it is deduced that no nucleic acids or a quantity of nucleic acid below detection level is present in the biological sample. Besides, the cycle of the PCR or RT-PCR at which the signal higher than background signal intensity is measured is named the threshold cycle (CT). It is well known to one of skill in the art that CT values are proportional to the log₁₀ of the starting amounts of nucleic acids in the biological sample Accordingly, the quantity of nucleic acids present, i.e. the nucleic acid load of the biological sample, can be readily determined, if necessary by reference to a standard curve.

Numerous real-time PCR and RT-PCR techniques, generally differing in the signal generation system and the labelled probes used, can be used according to the invention, such as the so-called Taqman or Molecular Beacons assays. However it is preferred that the real-time PCR or RT-PCR according to the invention is of the Taqman type. Taqman type real-time PCR or RT-PCR is well known to one of skill in the art and has been originally described in 1991 by Holland et al. (1991) Proc. Natl. Acad. Sci. USA 88:7276-7280.

Briefly, TaqMan probes consist of a fluorophore covalently attached to the 5'-end of an oligonucleotide probe and a quencher at the 3'-end. The probe is such that the quencher molecule quenches the fluorescence emitted by the fluorophore when excited by a thermocycler's light source via FRET (Fluorescence Resonance Energy Transfer). Thus, as long as the fluorophore and the quencher are in proximity, *i.e*. are attached to the probe, quenching inhibits any fluorescence signals. Besides, TaqMan probes are designed such that they anneal to a target within amplified DNA region. As the thermostable polymerase used for performing PCR or RT-PCR extends the primer and synthesizes the nascent strand, its 5' to 3' exonuclease activity degrades the probe that has annealed to the template. Degradation of the probe releases the fluorophore from it and breaks the close proximity to the quencher, thus relieving the quenching effect and allowing fluorescence of the fluorophore. Hence, fluorescence detected in the quantitative PCR thermal cycler is directly proportional to the fluorophore released and the amount of DNA template present in the PCR.

Preferably, the real-time PCR or RT-PCR according to the invention comprise the following thermocycling conditions:
- optionally 10 min at 50°C for reverse transcription, followed by
- 5 min at 95°C, followed by
- 50 cycles of 95°C for 15 s and 60°C for 1 min.

Advantageously, the above-defined thermocycling conditions are similar to that used by the real-time RT-PCR generic HIV-1 RNA assay described by Rouet et al. (2005) J. Clin. Microbiol. 43:2709-17 and commercialized by Biocentric, which is currently used with success in many resource-limited countries. Accordingly, the method for detecting or quantifying Human Immunodeficiency Virus-2 (HIV-2) nucleic acids in a biological sample according to the invention can be used on the same thermocycler, with the same software program and even, if necessary, in the same amplification plate, as biological samples intended for HIV-1 nucleic acid detection or quantification.

### Primers and probes

As intended herein a primer is an oligonucleotide, preferably a DNA oligonucleotide, useful to prime replication by a DNA polymerase, in particular a thermostable DNA polymerase, or reverse transcription by a reverse transcriptase. Preferably, primers according to the invention comprise no more than 50 nucleotides, more preferably no more than 40 nucleotides, and most preferably no more than 30 nucleotides.

As intended herein a probe is an oligonucleotide, preferably a DNA oligonucleotide, which can anneal to DNA molecules amplified as a result of the PCR or the RT-PCR according to the invention. Preferably, probes according to the invention comprise no more than 50 nucleotides, more preferably no more than 40 nucleotides, and most preferably no more than 30 nucleotides. The labelled probe is such that a detectable signal, preferably fluorescence, is emitted and increases in intensity as a consequence of the accumulation of amplified DNA molecules. More preferably the probes according to the invention are labelled, in particular covalently labelled, by a flurophore and by a quencher, the fluorophore-quencher pair being such that the quencher quenches fluorescence emission by the fluorophore. In this frame, it is preferred that the fluorophore is attached at or near the 5' end of the probe and that the quencher is attached at or near the 3' end of the probe. Numerous suitable fluorophore-quencher pairs according to the invention can be devised by one of skill in the art. By way of example, the fluorophore-quencher pairs 6-carboxyfluorescein (FAM)-carboxytetramethylrhodamine (TAMRA) and 6-carboxyfluorescein (FAM)-Black Hole Quencher-1 (BHQ1) are suitable for the labelled probes according to the invention. A particularly preferred fluorophore-quencher pair according to the invention is 6-carboxyfluorescein (FAM)-Black Hole Quencher-1 (BHQ1). Besides it is also preferred that the 2 labelled probes according to the invention are labelled by a same fluorophore-quencher pair. Thus, it is particularly preferred that the 2 labelled probes are labelled with 6-carboxyfluorescein (FAM) at their 5'end and with Black Hole Quencher-1 (BHQ1) at their 3' end.

As intended herein, where a primer or a probe according to the invention is said to "comprise" a particular sequence, the primer or the probe may also comprise additional sequences extending from the 5' end and/or 3' end of the particular sequence. In contrast, where a primer or a probe according to the invention "consists of" a particular sequence, the primer or the probe does not comprise supplementary sequences in addition to the particular sequence.

Preferably, the at least 4 primers according to the invention respectively consist of sequences SEQ ID NO: 1, SEQ ID NO: 2, SEQ ID NO: 4 and SEQ ID NO: 5, and the 2 labelled probes according to the invention respectively consist of sequences SEQ ID NO: 3 and SEQ ID NO: 6.

As intended herein, a "sequence having at least 90% identity to SEQ ID NO: X", in particular differs from SEQ ID NO: X by the insertion, the suppression or the substitution of at least one nucleotide. Besides, the percentage of identity between two nucleotide sequences is defined herein as the number of positions for which the bases are identical when the two sequences are optimally aligned, divided by the total number of bases of the longest of the two sequences. Two sequences are said to be optimally aligned when the percentage of identity is maximal. Besides, as will be clear to one of skill in the art, it may be necessary to add gaps in order to obtain an optimal alignment between the two sequences.

Preferably, a sequence according to the invention having at least 90% identity to SEQ ID NO: 1, 2, 3, 4, 5, 6 or 7 has respectively at least 95%, more preferably at least 98% identity with SEQ ID NO: 1, 2, 3, 4, 5, 6 or 7.

The primers according to the invention comprising sequences SEQ ID NO: 1 and SEQ ID NO: 2 or sequences having at least 90% identity to SEQ ID NO: 1 and SEQ ID NO: 2 and the probe comprising or consisting of sequence SEQ ID NO: 3, a sequence complementary to SEQ ID NO: 3, or a sequence having at least 90% identity to SEQ ID NO: 3 or the complementary thereof, are useful to detect a portion of the LTR region of HIV-2 genome.

The primers according to the invention comprising sequences SEQ ID NO: 4 and SEQ ID NO: 5 or sequences having at least 90% identity to SEQ ID NO: 4 and SEQ ID NO: 5 and the probe comprising or consisting of sequence SEQ ID NO: 6, a sequence complementary to SEQ ID NO: 6, or a sequence having at least 90% identity to SEQ ID NO: 6 or the complementary thereof, are useful to detect a portion of the Gag region of HIV-2 genome.

The primer according to the invention comprising or consisting of sequence SEQ ID NO: 7 or a sequence having at least 90% identity to SEQ ID NO: 7 is useful to improve the detection and quantification of subtype B HIV-2 nucleic acids.

As will be clear to one of skill in the art and by way of example, the sequence complementary to a sequence 5' TAGGTTACGGCCCGGCGGAAAGA 3' (SEQ ID NO: 6) is 5' TCTTTCCGCCGGGCCGTAACCTA 3' (SEQ ID NO: .9)

Besides, as will also be clear to one of skill in the art SEQ ID NO: 3 (CTTGGCCGGYRCTGGGCAGA) is a so-called degenerated sequence wherein Y represents C and T, and R represents A and G.

Advantageously, the use of primers and probes according to the invention in real-time PCR and RT-PCR (i) provide for the specific detection and quantification of HIV-2 nucleic acids over HIV-1 nucleic acids, (ii) lower the detection and quantification limit of the prior art assays, and (iii) provide for an improved detection and quantification of subtype B HIV-2 nucleic acids.

### Biological sample

As intended herein a "biological sample" relates to any sample taken from a human individual liable to contain HIV-2 nucleic acids, such as a blood sample, a plasma sample, or a seminal sample. As will be clear to one of skill in the art the biological sample may have been treated after having been taken from the individual and before being submitted to a real-time PCR or RT-PCR according to the invention. Such treatments notably encompass centrifugation, treatment by an anticoagulant, such as EDTA, or nucleic acid concentration, purification or extraction. Thus, by way of example, a biological sample according to the invention may be a nucleic acid solution extracted from a sample, such as plasma sample, taken from a human individual.

### Kit and mix

As intended herein in a "kit" according to the invention, one or more of the components of the kit may be packaged or compartmented separately from the rest of the components.

As intended herein in a "mix" according to the invention, all the components of the mix are in a single compartment. Preferably, the mix according to the invention is a PCR mix or a RT-PCR mix.

Additional reagents to the primers and probes according to the invention can be easily devised by one of skill in the art and notably encompass a thermostable DNA polymerase, a reverse transcriptase, dNTPs, salts, in particular Mn²⁺ and Mg²⁺ salts, and buffers.

The present invention will be further described by the following non-limiting figures and example.

### Description of the figures

Figure 1

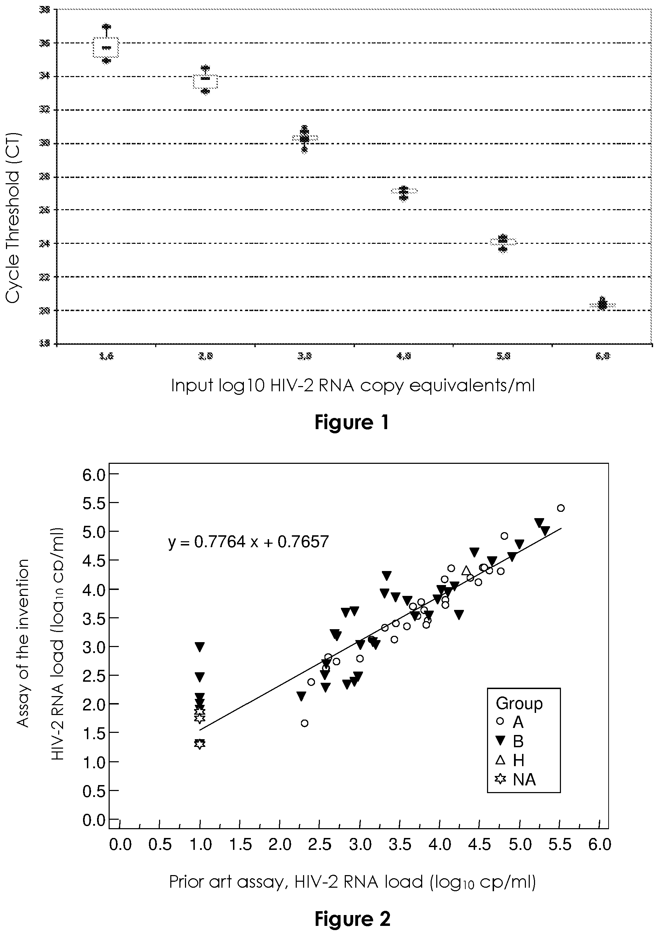

   Figure 1 represents the standard curve of the HIV-2 RNA real-time viral load assay according to the invention. The cycle threshold (CT) (vertical axis) is the number of cycles at which fluorescence passes a fixed limit (time to positivity) and the input log₁₀ HIV-2 copy equivalents/ml (horizontal axis) represents the standardization viral load present in the sample submitted to real-time RT-PCR. Median values and 25% and 75% interquartile ranges (box plot) of the CT are indicated. The vertical lines show the ranges of the CT.
Figure 2
   Figure 2 is a scatter diagram of the HIV-2 RNA load (expressed as log₁₀ copies/ml) as determined by the assay according to the invention (vertical axis) vs. as determined by the prior art assay (horizontal axis), according to the genetic group (A, B, H or non genotypable (NA)) of the 78 detectable (0 to <40 cp/mL) or quantifiable samples (r2 =0.8812). An arbitrary value of 10 cp/ml was attributed to samples undetectable in the current assay, and 20 cp/ml to those detectable in the new assay (0 to <40 cp/mL).
Figures 3 and 4

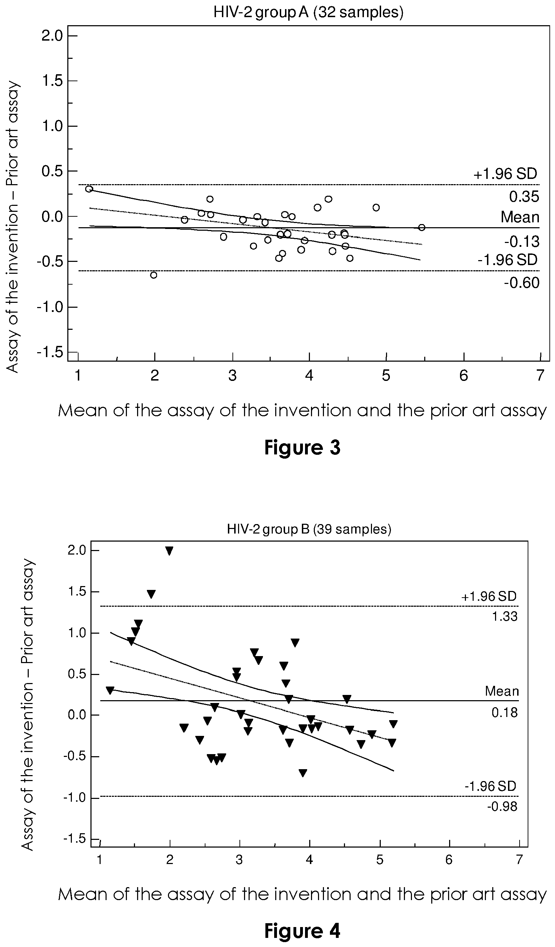

   Figures 3 and 4 represent Bland and Altman curves for measuring the degree of agreement in log₁₀ cp/ml between the assay according to the invention and the prior art assay; for group A (N=32) (Figure 3) and group B (N=39) (Figure 4) samples detectable (0 to 40 cp/mL) and quantifiable (>40 cp/mL) with the assay according to the invention. An arbitrary value of 10 cp/ml was attributed to samples undetectable with the prior art assay, and 20 cp/ml to those detectable with the assay according to the invention (between 0 and 40 cp/ml).

### EXAMPLE

### Materials and Methods

### HIV-2 RNA assay according to the invention

The test is based on a one-step duplex Taqman PCR approach targeting a conserved consensus region in the long terminal repeat (*LTR*) region and the *Gag* region.

The forward and reverse primers for the *LTR* region are 5'-TCTTTAAGCAAGCAAGCGTGG-3' (SEQ ID NO: 1) and 5'-AGCAGGTAGAGCCTGGGTGTT-3' (SEQ ID NO: 2), respectively (Rouet et al. (2004) J. Clin. Microbiol. 42:4147-53), with an internal probe (5' FAM-CTTGGCCGGYRCTGGGCAGA-BHQ1 3', SEQ ID NO: 3) modified to optimize efficiency for HIV-2 group B.

The forward and reverse primers for the Gag region are F3 5'-GCGCGAGAAACTCCGTCTTG-3' (SEQ ID NO: 4) and R1 5'-TTCGCTGCCCACACAATATGTT-3' (SEQID NO: 5), respectively (Damond et al. (2005) J. Clin. Microbiol. 43:4234-6), and the internal HIV-2 Taqman gag probe is S65GAG2 5' FAM-TAGGTTACGGCCCGGCGGAAAGA-BHQ1 3' (Eurogentec, Seraing, Belgium) (Damond et al. (2005) J. Clin. Microbiol. 43:4234-6).

**Table 1: Primers and probes used for RT-PCR analysis**

| **Primers & Probes** | ***LTR*** | ***Gag*** |
|---|---|---|
| **Forward primer** | 5'-TCTTTAAGCAAGCAAGCGTGG-3' (SEQ ID NO: 1) | 5'-GCGCGAGAAACTCCGTCTTG-3' (SEQ ID NO: 4) |
| **Reverse primer** | 5'-AGCAGGTAGAGCCTGGGTGTT-3' (SEQ ID NO: 2) | 5'-TTCGCTGCCCACACAATATGTT-3' (SEQ ID NO: 5) |
| **Probe** | 5'-CTTGGCCGGYRCTGGGCAGA-3' (SEQ ID NO: 3) | 5'-TAGGTTACGGCCCGGCGGAAAGA-3' (SEQ ID NO: 6) |

RNA was extracted from 200 µl of plasma by using the QIAamp viral RNA mini kit (Qiagen, Courtaboeuf, France), as in the Biocentric generic HIV-1 charge virale assay, in laboratories A and B (Necker Hospital, Paris, and Charles Nicolle Hospital, Rouen) or from 1 ml with the Total NA large volume MagnaPure kit (Roche Automated System, Meylan, France) in laboratory C (Bichat Claude Bernard Hospital, Paris).

The reaction mix consists of a 20-µL volume containing the RNA extract (10 µL), primers (500 nM each), probes (250 nM each), and 1X PCR buffer (4X One-step mix, Invitrogen, Cergy Pontoise, France).

The thermocycling conditions are those used for the Biocentric HIV-1 assay: 10 min at 50°C and 5 min at 95°C, followed by 50 cycles of 95°C for 15 s and 60°C for 1 min. Amplification and data acquisition are carried out with the TaqMan ABI realtime PCR system (Applied Biosystems, Courtaboeuf, France). The log₁₀ number of targets initially present is proportional to the cycle threshold (CT) and is determined from the standard curve.

A BIOQ HIV-2 RNA group A quantification panel (P0182; Rijswijk, The Netherlands) was used as the external standard. The standard, evaluated at 2.93x10⁶ cp/ml, was first diluted in RPMI culture medium to a theoretical concentration of 1 000 000 cp/ml (2 400 000 IU/ml), followed by serial 10-fold dilution to concentrations ranging from 1 000 000 (5 log₁₀) to 100 cp/ml (2 log₁₀), and a final dilution to 40 cp/ml (1.6 log₁₀).

### Determination of the analytic performance of the assay according to the invention

Specificity was determined by testing plasma samples from 49 HIV-negative subjects and 30 HIV-1 group M-positive patients with viral loads ranging from >20 to <10 000 000 cp/mL. Nine HIV-1 group O coculture supernatants were also tested.

Linearity was assessed using the BIOQ external standard diluted in RPMI to 1 000 000, 100 000, 10 000, 1000, 100 and 40 cp/ml (each tested in 10 runs at Lab A).

Analytical sensitivity was determined by dilution in RPMI of the BIOQ external standard to 100, 50, 40, 20 and 10 cp/mL (10 replicates each).

To determine within-run reproducibility, the BIOQ external standard was tested at concentrations of 10 000 and 100 cp/mL in each of the three laboratories (10 replicates for each dilution).

To determine between-run reproducibility, an HIV-2 positive control was prepared by serial dilution in HIV-negative EDTA human plasma of a coculture supernatant of an HIV-2 group A isolate (Genbank accession number AY688870, SEQ ID NO: 8). This solution was diluted to obtain aliquots with theoretical concentrations ranging from 10 000 to 100 000 cp/mL in the current assay. These aliquots were each tested once in 7 separate runs with the Magna pure automated extraction system (Lab C) and in respectively 18 and 7 separate runs with Qiagen manual extraction at Lab A and Lab B.

### Statistical analysis

MedCalc software (Ostend, Belgium) was used for data analysis. Bland and Altman curves were used to represent the degree of agreement between the two techniques (Bland & Altman (1986) Lancet 1:307-10). The X-axis bore the mean values for each sample obtained with the two techniques, and the Y-axis the difference between the values obtained with the two techniques.

Disagreement between the two techniques was defined as a difference of more than 0.5 log₁₀ for a given sample.

### Clinical samples

One hundred plasma samples from HIV-2-infected patients (n=100) included in the French National HIV-2 Cohort (ANRS CO05) were selected according to the viral genotype and the HIV-2 RNA concentration, as determined with the technique described in Damond et al. (2005) J. Clin. Microbiol. 43:4234-6. The HIV-2 group was determined for 89 samples, as previously described (Damond et al. (2004) AIDS Res Hum Retroviruses 20:666-672 and Plantier et al. (2004) J. Clin. Microbiol. 42:5866-70): 38 samples were group A, 50 group B, and one group H. Genotyping was not available for the remaining 11 samples, owing to the absence of detectable RNA and a lack of whole blood or mononuclear cells for viral DNA assay.

The selected samples had the following characteristics: <100 (2 log₁₀) cp/ml (n=39, 9 group A and 19 group B, 11 non genotypable), 100 (2 log₁₀) - 1000 (3 log₁₀) cp/ml (n=16, 5 A and 11 B), 1000 (3 log₁₀) - 10 000 (4 log₁₀) (n=22, 12 A and 10 B), 10 000 (4 log₁₀) - 100 000 (5 log₁₀) (n=19, 11 A, 7 B and 1 H) and >100 000 (5 log₁₀) (n=4, 1 A and 3B).

### Results

### Analytic performances of the assay according to the invention

As expected, given the wide genomic divergence between HIV-1 and HIV-2, the HIV-2 primers did not hybridize to HIV-1 genes: all HIV-1-positive plasma samples and all HIV-negative samples were negative in the new assay, giving a specificity of 100%.

The standard curve showed a strong linear relationship between the CT values and log₁₀ HIV-2 RNA cp/mL (**Figure 1**). The median correlation coefficient was 0.9947 (range, 0.9831 to 0.9997), and the median slope was -3.37 (range, -3.16 to -3.62).

The analytical sensitivity of the assay was 100% at 40 cp/ml (1.6 log₁₀ cp/mL) and 90% at 20 cp/mL (1.3 log₁₀cp/mL) after Roche Magna pure automated extraction of 1 mL, and 100% at 50 cp/mL (1.7 log₁₀ cp/mL) after manual extraction of 200 µL. Optimization of the assay sensitivity after manual extraction was evaluated using 1 mL of plasma: the sample was centrifuged at 17 000 rpm and the pellet was resuspended in 200 µL of RPMI medium prior to manual extraction, with elution in 60 µL. This yielded 90% sensitivity at 10 cp/mL (1 log₁₀ cp/mL).

Within-run reproducibility was evaluated in the three labs by using the BIOQ external standard with theoretical virus concentrations of 10 000 and 100 cp/mL (4 and 2 log₁₀ cp/mL): for the 4 log₁₀ cp/mL value we obtained a mean of 3.91 log₁₀ cp/mL at Lab C, 4.1 log₁₀ cp/mL at Lab A and 4.2 log₁₀ cp/mL at Lab B, with within-run coefficients of variation of 1.61%, 0.54% and 1.10%, respectively. At the concentration of 2 log₁₀ cp/mL, the inventors obtained mean values of 2.03 log₁₀ cp/mL at Lab B, 2.07 log₁₀ cp/mL at Lab A, and 2.17 log₁₀ cp/mL at Lab C, with within-run coefficients of variation of 10.72%, 14.32% and 7.24%, respectively.

In between-run assays, the positive control with a theoretical concentration between 10 000 (4 log₁₀) and 100 000 cp/mL (5 log₁₀) was evaluated at 4.61 log₁₀ cp/mL in Lab C, 4.70 log₁₀ cp/mL in Lab A, and 4.88 log₁₀ cp/mL in Lab B, with coefficients of variation of 2.28%, 6.43% and 3.03%, respectively.

### Clinical performances

The clinical performances of the new assay were evaluated in Lab C. Clinical samples of 1 ml were extracted with the automated MagnaPure method then tested in parallel with the ABI device for the assay according to the invention and the Light Cycler 1.5 device for the prior art assay described by Damond et al. (2005) J. Clin. Microbiol. 43:4234-6. The results obtained with the assay according to the invention were categorized into four groups (**Table 2**): undetectable (<40 cp/mL), detectable but not quantifiable (0 to <40 cp/mL), quantifiable between 40 and 100 cp/mL, and above the lower limit of quantification of the current assay (100 cp/mL).

Of the 39 samples below the quantification limit of 100 cp/mL in the current assay, 22 samples (56%) were also undetectable with the assay according to the invention (**Table 2**), while 10 samples (26%; 3 A, 3 B and 4 non genotypable) were detected at values between 0 and 40 cp/mL (range: 1 to 36 cp/mL). Three samples (7.7%; 1 B, 2 non genotypable) were quantified between 40 and 100 cp/mL (range: 56 to 79 cp/mL), and four samples (10%; all B) were quantified above 100 cp/mL (range: 102 to 970 cp/mL); the latter corresponded to true false-negative samples, taking into account the 100 cp/mL cut-off of the current assay.

These results showed that the test improved the detection or quantification of 17/39 samples (43.6%), including eight group B samples (**Table 1**).

All 61 plasma samples with values above 100 cp/mL in the prior art assay were detectable with the test according to the invention. One sample at 209 cp/mL (2.32 log₁₀ cp/ml) in the current assay gave a value of 46 cp/mL (1.69 log₁₀ cp/ml) in the test according to the invention (**Table 1**).

A scatter plot was constructed for the 78 samples that were detectable or quantifiable with the assay according to the invention and quantifiable with the pior art assay (**Figure 2**). It showed a wider dispersion of values for quantifiable group B samples than for quantifiable group A samples, as well as better detection or quantification of group B and non genotypable samples. This was confirmed by scatter equations specific for group A samples (n=32; y = 0.8921x + 0.2545, r2 = 0.9569) and group B samples (n=39; y = 0.7136x + 1.0484, r2 = 0.8067), and also by Bland-Altman representations (**Figure 3**). Homogeneous quantification (+/-1.96 SD, range from -0.35 to 0.6) and similar values (median difference of -0.13) were obtained with the assay according to the invention and the prior art assay for group A samples. The median difference between the two assays for group B samples was +0.18, but with greater heterogeneity (+/-1.96 SD, range -1.33 to 0.98).

The only HIV-2 group H sample gave very similar results with the two assays (4.33 log₁₀ and 4.34 log₁₀).

### Conclusion

HIV-2 infection differs markedly from HIV-1 infection, notably by its slower natural course, different therapeutic management, and genetic diversity. Specific molecular methods are therefore necessary for diagnosis and patient monitoring. Current assays, mainly consisting of in-house methods or unvalidated derivatives of commercial kits, suffer from major limitations in terms of their sensitivity, accuracy, and coverage of HIV-2 genetic diversity.

The aim of this work was to develop a quantitative assay that takes into account both the low viral load seen in most HIV-2-infected patients and the broad genetic diversity of HIV-2, especially group B. In addition, as most cases of HIV-2 infection occur in West Africa, such a test must be affordable and easy to implement in developing countries, as previously achieved with the generic HIV-1 viral load assay marketed by Biocentric.

The inventors improved the assay currently used to monitor the French HIV-2 cohort, which is based on amplification of the HIV-2 Gag region and has a lower limit of quantification of 100 cp/ml (Damond et al. (2005) J. Clin. Microbiol. 43:4234-6). The inventors also used the same operating conditions as those of the Biocentric HIV-1 assay kit, in order to facilitate its use either for HIV-2 alone or jointly for HIV-1 and HIV-2.

The new test exhibits good linearity (40 to 1 000 000 cp/ml) and within-run reproducibility (<15%). Its inter-laboratory reproducibility was validated by evaluation at three different sites. Both manual and automated extraction methods were validated, for compatibility with local practices in resource-limited countries.

Relative to the prior art assay of Damond et al. (2005) J. Clin. Microbiol. 43:4234-6, the test according to the invention has a significantly better analytical limit of quantification, reaching 50 cp/ml with manual extraction of 200 µl of plasma and 40 cp/ml with automated extraction of 1 mL. Assuming a probit rate of 90%, the detection limit with 1 ml of plasma would be 10 cp/ml and 20 cp/ml, respectively. This very good analytical sensitivity matches that of recently published in-house methods (5, 11, 29) and is compatible with virological monitoring of HIV-2 infection, as more than 60% of untreated patients have viral loads below 250 cp/ml. The new test was able to detect and/or quantify more than one-third of samples that were undetectable with our current assay, which has a quantification limit of 100 cp/ml. This excellent sensitivity should prove useful both for pathophysiological studies and for treatment monitoring.

The most difficult issue facing the development of HIV-2 viral load assays is the genetic diversity of this virus (especially group B), some variants being under-quantified or escaping detection with current tests. Three teams recently reported improved sensitivity for HIV-2, but they mainly used supernatants (Delarue et al. (2013) J. Clin. Virol. 58:461-7) or a limited number of samples (Chang et al. (2012) J. Clin. Virol. 55:128-33, Styer et al. (2013) J. Clin. Virol. 58 Suppl 1:e127-33) or validated detection but not quantification (Styer et al. (2013) J. Clin. Virol. 58 Suppl 1:e127-33), leaving questions as to their clinical performance, especially for group B viruses.

The inventors evaluated the assay according to the invention on 100 clinical samples, 39% of which were undetectable with the comparative prior art assay described in Damond et al. (2005) J. Clin. Microbiol. 43:4234-6, representative of the molecular epidemiology of groups A and B, plus the only one divergent sample of group H. Half the samples corresponded to group B, and more than one-third of them (n=19) were undetectable with the comparative prior art assay. The inventors developed a duplex method capable of simultaneously amplifying the *LTR* and *Gag* regions, which unexpectedly resulted in a synergic improvement of the detection of group B viruses by reducing the risk of mismatches. The new and current HIV-2 assay methods gave similar results for the single group H sample and for the group A samples (although 3 additional group A samples were detectable with the new test), whereas the new test developed by Delarue et al gave values nearly 0.5 log₁₀ lower than their reference test (Delarue et al. (2013) J. Clin. Virol. 58:461-7). Eight additional group B samples (42%) were detected or quantified with our new test, four samples having values of 102 to 970 cp/mL. This unexpected improvement is due to the addition of primers in the *LTR* region and to changes in the *LTR* probe. However, the wider dispersion of values and the larger number of group B than group A samples with differences exceeding 0.5 log₁₀ relative to the current assay illustrate the greater difficulty of group B quantification. In addition, six non-genotypable samples were better detected or quantified with our new assay.

Advantageously, the test according to the invention can be used in the same operating conditions as the generic HIV-1 RNA assay currently used with success in many resource-limited countries (Rouet et al. (2005) J. Clin. Microbiol. 43:2709-17) meaning it can be used on the same machine, with the same software program and even, if necessary, in the same amplification plate, as HIV-1 samples. This will reduce analytical costs by increasing the number of samples per run.

The assay according to the invention has analytical performances at least equal to that of other newly developed tests (Chang et al. (2012) J. Clin. Virol. 55:128-33, Delarue et al. (2013) J. Clin. Virol. 58:461-7 and Styer et al. (2013) J. Clin. Virol. 58 Suppl 1:e127-33) as the performances of these tests have not been as thoroughly evaluated as the test according to the invention. Thus the test described by Chang *et al.*, adapted from the Abbott platform (Abbott Molecular, Chicago, II), was evaluated on few group B samples and was not compared with other techniques. Styer *et al.* recently compared their method with this "Abbott" technique and observed a difference of -0.35 log10 UI/mL, but they used a limited panel of uncharacterized samples, ruling out any evaluation of in terms of genetic diversity. Finally, Styer *et al.* and Delarue *et al.* used a two-step method, whereas the assay according to the invention is performed in a single step.

In conclusion, the inventors have developed and standardized an assay with better analytical sensitivity than the technique currently used to monitor HIV-2-infected patients in France. The assay according to the invention also has improved clinical sensitivity and has been validated on a broad, well-characterized sample panel, in contrast to recently published tests. The analytical performance of this new assay, which is easy to perform, makes it suitable for use in resource-limited countries in which multiple HIV-2 variants circulate. In addition, the assay according to the invention can be used on the same analytical platforms and in the same run as tests for HIV-1, thus improving its cost-efficiency for monitoring patients infected with HIV-1 and/or HIV-2. This possibility of simultaneous analysis will facilitate molecular diagnosis of mother-to-child transmission of HIV-1 and/or HIV-2, and also diagnosis and follow-up of dual HIV-1/HIV-2 infection in the same sample. Finally, use of this assay for virological monitoring will provide new insights into the natural history of HIV-2 infection at different clinical stages.

## Claims

1. A method for detecting or quantifying Human Immunodeficiency Virus-2 (HIV-2) nucleic acids in a biological sample, comprising:
a) performing a real-time polymerase chain reaction (PCR) or a real-time reverse transcriptase polymerase chain reaction (RT-PCR) on nucleic acids of the biological sample with:
(i) at least 4 primers respectively comprising or consisting of:
- sequence SEQ ID NO: 1 or a sequence having at least 90% identity to SEQ ID NO: 1, and
- sequence SEQ ID NO: 2 or a sequence having at least 90% identity to SEQ ID NO: 2, and
- sequence SEQ ID NO: 4 or a sequence having at least 90% identity to SEQ ID NO: 4, and
- sequence SEQ ID NO: 5 or a sequence having at least 90% identity to SEQ ID NO: 5,
wherein the primers comprise not more than 30 nucleotides, and
(ii) at least 2 labelled probes respectively comprising or consisting of:
- sequence SEQ ID NO: 3, a sequence complementary to SEQ ID NO: 3, or a sequence having at least 90% identity to SEQ ID NO: 3 or the complementary thereof, and
- sequence SEQ ID NO: 6, a sequence complementary to SEQ ID NO: 6, or a sequence having at least 90% identity to SEQ ID NO: 6 or the complementary thereof,
wherein the probes comprise not more than 30 nucleotides, and
b) determining therefrom the presence or absence and/or the quantity of HIV-2 nucleic acids in the biological sample.

2. The method according to claim 1, for detecting or quantifying HIV-2 RNA.

3. The method according to claim 1 or 2, wherein the at least 4 primers respectively consist of sequences SEQ ID NO: 1, SEQ ID NO: 2, SEQ ID NO: 4 and SEQ ID NO: 5, and the 2 labelled probes respectively consist of sequences SEQ ID NO: 3 and SEQ ID NO: 6.

4. The method according to any of claims 1 to 3, wherein the labelled probes are labelled with 6-carboxyfluorescein (FAM) at their 5'end and with Black Hole Quencher-1 (BHQ1) at their 3' end.

5. The method according to any of claims 1 to 4, wherein the PCR or RT-PCR comprises the following thermocycling conditions:
- optionally 10 min at 50°C for reverse transcription, followed by
- 5 min at 95°C, followed by
- 50 cycles of 95°C for 15 s and 60°C for 1 min.

6. The method according to any of claims 1 to 5, further comprising determining or quantifying HIV-1 nucleic acids in a biological sample.

7. A kit or a mix for detecting or quantifying HIV-2 nucleic acids, comprising:
a) at least 4 primers respectively comprising or consisting of:
- sequence SEQ ID NO: 1 or a sequence having at least 90% identity to SEQ ID NO: 1, and
- sequence SEQ ID NO: 2 or a sequence having at least 90% identity to SEQ ID NO: 2, and
- sequence SEQ ID NO: 4 or a sequence having at least 90% identity to SEQ ID NO: 4, and
- sequence SEQ ID NO: 5 or a sequence having at least 90% identity to SEQ ID NO: 5,
wherein the primers comprise not more than 30 nucleotides, and
b) at least 2 labelled probes respectively comprising or consisting of:
- sequence SEQ ID NO: 3, a sequence complementary to SEQ ID NO: 3, or a sequence having at least 90% identity to SEQ ID NO: 3 or the complementary thereof, and
- sequence SEQ ID NO: 6, a sequence complementary to SEQ ID NO: 6, or a sequence having at least 90% identity to SEQ ID NO: 6 or the complementary thereof,
wherein the probes comprise not more than 30 nucleotides, and
c) optionally additional reagents for performing PCR or RT-PCR.

8. The kit or the mix according to claim 7, wherein the at least 4 primers respectively consist of sequences SEQ ID NO: 1, SEQ ID NO: 2, SEQ ID NO: 4 and SEQ ID NO: 5, and the 2 labelled probes respectively consist of sequences SEQ ID NO: 3 and SEQ ID NO: 6.

9. The kit or the mix according to claim 7 or 8, wherein the labelled probes are labelled with 6-carboxyfluorescein (FAM) at their 5'end and with Black Hole Quencher-1 (BHQ1) at their 3' end.

10. The kit or the mix according to any of claims 7 to 9, further comprising primers and labelled probes for detecting or quantifying HIV-1 nucleic acids.

11. The use of the kit or the mix as defined in claims 7 to 10, for detecting or quantifying HIV-2 nucleic acids of a biological sample.

12. An *in vitro* method for diagnosing HIV-2 infection, or determining HIV-2 viral load, in an individual, comprising the steps of:
(a) performing the method for detecting or quantifying Human Immunodeficiency Virus-2 (HIV-2) nucleic acids in a biological sample taken from the individual as defined in claims 1 to 6;
(b) determining therefrom whether the individual is infected by HIV-2 or the HIV-2 viral load of the individual.

13. An *in vitro* method for determining whether an individual is liable to benefit from a treatment with antiretroviral therapy (ART) or from an adjustment of ART, comprising performing the method for diagnosing HIV-2 infection, or determining HIV-2 viral load, according to claim 12.

14. Nucleotide reverse transcriptase inhibitors (NRTIs), Protease inhibitors (Pis) and/or Integrase inhibitors for use in the prevention or treatment of HIV-2 infection in an individual, wherein the individual is determined to be liable to benefit from a treatment with ART or from an adjustment of ART by performing the method according to claim 13.

15. A probe comprising or consisting of sequence SEQ ID NO: 3, or a sequence complementary to SEQ ID NO: 3.

## Patentansprüche

1. Verfahren zum Nachweis oder zur Quantifizierung von Nukleinsäuren des Menschlichen Immunschwächevirus 2 (HIV-2) in einer biologischen Probe, umfassend:
a) Ausführung einer Echtzeit-Polymerasekettenreaktion (PCR) oder einer Echtzeit-Reverse-Transkriptase-Polymerasekettenreaktion (RT-PCR) an Nukleinsäuren der biologischen Probe mit
(i) mindestens 4 Primern, jeweils umfassend oder bestehend aus:
- Sequenz SEQ ID NO: 1 oder eine zu mindestens 90% mit SEQ ID NO: 1 identische Sequenz, und
- Sequenz SEQ ID NO: 2 oder eine zu mindestens 90% mit SEQ ID NO: 2 identische Sequenz, und
- Sequenz SEQ ID NO: 4 oder eine zu mindestens 90% mit SEQ ID NO: 4 identische Sequenz, und
- Sequenz SEQ ID NO: 5 oder eine zu mindestens 90% mit SEQ ID NO: 5 identische Sequenz, und
wobei die Primer nicht mehr als 30 Nukleotide umfassen, und
(ii) mindestens 2 markierte Sonden, jeweils umfassend oder bestehend aus:
- Sequenz SEQ ID NO: 3, eine zu SEQ ID NO: 3 komplementäre Sequenz, oder eine zu mindestens 90% mit SEQ ID NO: 3 identische Sequenz oder die hierzu komplementäre Sequenz, und
- Sequenz SEQ ID NO: 6, eine zu SEQ ID NO: 6 komplementäre Sequenz, oder eine zu mindestens 90% mit SEQ ID NO: 6 identische Sequenz oder die hierzu komplementäre Sequenz,
wobei die Sonden nicht mehr als 30 Nukleotide umfassen, und
b) daraus Bestimmung der An- oder Abwesenheit von und/oder Quantifizierung von HIV-2-Nukleinsäuren in der biologischen Probe.

2. Verfahren nach Patentanspruch 1 zum Nachweis oder zur Quantifizierung von HIV-2-RNA.

3. Verfahren nach Patentanspruch 1 oder 2, in dem die mindestens 4 Primer aus SEQ ID NO: 1, SEQ ID NO: 2, SEQ ID NO: 4 bzw. SEQ ID NO: 5 bestehen, und die 2 markierten Sonden aus den Sequenzen SEQ ID NO: 3 bzw. SEQ ID NO: 6 bestehen.

4. Verfahren nach irgendeinem der Patentansprüche 1 bis 3, in dem die markierten Sonden mit 6-Carboxyfluorescein (FAM) an ihrem 5'-Ende markiert sind und mit Black Hole Quencher-1 (BHQ1) an ihrem 3'-Ende.

5. Verfahren nach irgendeinem der Patentansprüche 1 bis 4, in dem die PCR oder RT-PCR die folgenden Wärmezyklusbedingungen aufweist:
- optional 10 min bei 50°C für die reverse Transkription, gefolgt von
- 5 min bei 95°C, gefolgt von
- 50 Zyklen bei 95°C für 15 s und 60°C für 1 min.

6. Verfahren nach irgendeinem der Patentansprüche 1 bis 5, außerdem den Nachweis oder die Quantifizierung von HIV-1-Nukleinsäuren in einer biologischen Probe umfassend.

7. Satz oder Gemisch zum Nachweis oder zur Quantifizierung von HIV-2-Nukleinsäuren, umfassend:
a) mindestens 4 Primer, jeweils umfassend oder bestehend aus:
- Sequenz SEQ ID NO: 1 oder eine zu mindestens 90% mit SEQ ID NO: 1 identische Sequenz, und
- Sequenz SEQ ID NO: 2 oder eine zu mindestens 90% mit SEQ ID NO: 2 identische Sequenz, und
- Sequenz SEQ ID NO: 4 oder eine zu mindestens 90% mit SEQ ID NO: 4 identische Sequenz, und
- Sequenz SEQ ID NO: 5 oder eine zu mindestens 90% mit SEQ ID NO: 5 identische Sequenz,
wobei die Primer nicht mehr als 30 Nukleotide umfassen, und
b) mindestens 2 markierte Sonden, jeweils umfassend oder bestehend aus:
- Sequenz SEQ ID NO: 3, eine zu SEQ ID NO: 3 komplementäre Sequenz, oder eine zu mindestens 90% mit SEQ ID NO: 3 identische Sequenz oder die hierzu komplementäre Sequenz, und
- Sequenz SEQ ID NO: 6, eine zu SEQ ID NO: 6 komplementäre Sequenz, oder eine zu mindestens 90% mit SEQ ID NO: 6 identische Sequenz oder die hierzu komplementäre Sequenz,
wobei die Sonden nicht mehr als 30 Nukleotide aufweisen, und
c) optional zusätzliche Reagenzien zur Ausführung der PCR oder RT-PCR.

8. Satz oder Gemisch nach Patentanspruch 7, in dem die mindestens 4 Primer aus SEQ ID NO: 1, SEQ ID NO: 2, SEQ ID NO: 4 bzw. SEQ ID NO: 5 bestehen, und die 2 markierten Sonden aus den Sequenzen SEQ ID NO: 3 bzw. SEQ ID NO: 6 bestehen.

9. Satz oder Gemisch nach Patentanspruch 7 oder 8, in dem die markierten Sonden mit 6-Carboxyfluorescein (FAM) an ihrem 5'-Ende markiert sind und mit Black Hole Quencher-1 (BHQ1) an ihrem 3'-Ende.

10. Satz oder Gemisch nach irgendeinem der Patentansprüche 7 bis 9, außerdem Primer und Sonden zum Nachweis oder zur Quantifizierung von HIV-1-Nukleinsäuren umfassend.

11. Gebrauch des Satzes oder Gemisches nach irgendeinem der Patentansprüche 7 bis 10 zum Nachweis oder zur Quantifizierung von HIV-2-Nukleinsäuren in einer biologischen Probe.

12. *In vitro*-Verfahren zur Diagnose einer HIV-2-Infektion oder zur Bestimmung der HIV-2-Viruslast bei einem Patienten, folgende Schritte umfassend:
(a) Ausführung des Verfahrens zum Nachweis oder zur Quantifizierung von Nukleinsäuren des Menschlichen Immunschwächevirus 2 (HIV-2) in einer biologischen Probe, die dem Patienten abgenommen wurde, nach den Patentansprüchen 1 bis 6,
(b) daraus Bestimmung, ob der Patient mit HIV-2 infiziert ist, oder Bestimmung der HIV-2-Viruslast des Patienten.

13. *In vitro*-Verfahren zur Ermittlung, ob ein Patient aus einer Behandlung durch Antiretrovirentherapie (ART) wahrscheinlich Nutzen ziehen kann oder aus einer Anpassung einer ART, das Verfahren zur Diagnose einer HIV-2-Infektion oder zur Bestimmung der HIV-2-Viruslast nach Patentanspruch 12 umfassend.

14. Nukleotid-Reverse-Transkriptase-Hemmer (NRTI), Proteasehemmer (PI) und oder Integrasehemmer zum Gebrauch in der Vorbeugung und Behandlung von HIV-2-Infektionen eines Patienten, bei der bestimmt wird, ob der Patient wahrscheinlich aus einer Behandlung durch ART Nutzen zu ziehen kann oder aus einer Anpassung einer ART durch Ausführung des Verfahrens nach Patentanspruch 13.

15. Sonde, die Sequenz SEQ ID NO: 3 oder eine zu SEQ ID NO: 3 komplementäre Sequenz enthaltend oder daraus bestehend.

## Revendications

1. Procédé de détection ou de quantification d'acides nucléiques du virus de l'immunodéficience humaine-2 (VIH-2) dans un échantillon biologique, comprenant :
a) effectuer une réaction en chaîne par polymérase (PCR) en temps réel ou une réaction en chaîne par polymérase avec une transcriptase inverse (RT-PCR) en temps réel sur des acides nucléiques de l'échantillon biologique avec :
(i) au moins 4 amorces comprenant ou consistant respectivement en :
- une séquence SEQ ID NO : 1 ou une séquence ayant au moins 90% d'identité avec la SEQ ID NO : 1, et
- une séquence SEQ ID NO :2 ou une séquence ayant au moins 90% d'identité avec la SEQ ID NO :2, et
- une séquence SEQ ID NO :4 ou une séquence ayant au moins 90% d'identité avec la SEQ ID NO : 4, et
- une séquence SEQ ID NO : 5 ou une séquence ayant au moins 90% d'identité avec la SEQ ID NO : 5,
dans lequel les amorces ne comprennent pas plus de 30 nucléotides, et
(ii) au moins 2 sondes marquées comprenant ou consistant respectivement en :
- une séquence SEQ ID NO : 3, une séquence complémentaire à la SEQ ID NO : 3, ou une séquence ayant au moins 90% d'identité avec la SEQ ID NO : 3 ou la complémentaire de celle-ci, et
- une séquence SEQ ID NO : 6, une séquence complémentaire à la SEQ ID NO : 6, ou une séquence ayant au moins 90% d'identité avec la SEQ ID NO : 6 ou la complémentaire de celle-ci,
dans lequel les sondes ne comprennent pas plus de 30 nucléotides, et
b) déterminer à partir de celles-ci la présence ou l'absence et/ou la quantité d'acides nucléiques du VIH-2 dans l'échantillon biologique.

2. Procédé selon la revendication 1, pour détecter ou quantifier l'ARN du VIH-2.

3. Procédé selon la revendication 1 ou 2, dans lequel les au moins 4 amorces sont constituées respectivement des séquences SEQ ID NO : 1, SEQ ID NO : 2, SEQ ID NO : 4 et SEQ ID NO : 5, et les 2 sondes marquées sont constituées respectivement des séquences SEQ ID NO : 3 et SEQ ID NO : 6.

4. Procédé selon l'une quelconque des revendications 1 à 3, dans lequel les sondes marquées sont marquées avec la 6-carboxyfluorescéine (FAM) en leur extrémité 5'et avec le Black Hole Quencher-1 (BHQ1) en leur extrémité 3'.

5. Procédé selon l'une quelconque des revendications 1 à 4, dans lequel la PCR ou la RT-PCR comprend les conditions de thermocycleur suivantes :
- éventuellement 10 min à 50°C pour la transcription inverse, suivis de
- 5 min à 95°C, suivis de
- 50 cycles de 95°C pendant 15 s et 60°C pendant 1 min.

6. Procédé selon l'une quelconque des revendications 1 à 5, comprenant en outre la détermination ou la quantification d'acides nucléiques du VIH-1 dans un échantillon biologique.

7. Kit ou mélange pour détecter ou quantifier les acides nucléiques du VIH-2, comprenant :
a) au moins 4 amorces comprenant ou consistant respectivement en :
- une séquence SEQ ID NO : 1 ou une séquence ayant au moins 90% d'identité avec la SEQ ID NO : 1, et
- une séquence SEQ ID NO : 2 ou une séquence ayant au moins 90% d'identité avec la SEQ ID NO : 2, et
- une séquence SEQ ID NO : 4 ou une séquence ayant au moins 90% d'identité avec la SEQ ID NO ; 4, et
- une séquence SEQ ID NO : 5 ou une séquence ayant au moins 90% d'identité avec la SEQ ID NO:5,
dans lequel les amorces ne comprennent pas plus de 30 nucléotides, et
b) au moins 2 sondes marquées comprenant ou consistant respectivement en :
- une séquence SEQ ID NO : 3, une séquence complémentaire à la SEQ ID NO : 3, ou une séquence ayant au moins 90% d'identité avec la SEQ ID NO : 3 ou la complémentaire de celle-ci, et
- une séquence SEQ ID NO : 6, une séquence complémentaire à la SEQ ID NO : 6, ou une séquence ayant au moins 90% d'identité avec la SEQ ID NO : 6 ou la complémentaire de celle-ci,
dans lequel les sondes ne comprennent pas plus de 30 nucléotides, et
c) éventuellement des réactifs supplémentaires pour effectuer la PCR ou la RT-PCR.

8. Kit ou mélange selon la revendication 7, dans lequel les au moins 4 amorces sont constituées respectivement des séquences SEQ ID NO : 1, SEQ ID NO : 2, SEQ ID NO : 4 et SEQ ID NO : 5, et les 2 sondes marquées sont constituées respectivement des séquences SEQ ID NO : 3 et SEQ ID NO : 6.

9. Kit ou mélange selon la revendication 7 ou 8, dans lequel les sondes marquées sont marquées avec la 6-carboxyfluorescéine (FAM) en leur extrémité 5'et avec le Black Hole Quencher-1 (BHQ1) en leur extrémité 3'.

10. kit ou mélange selon l'une quelconque des revendications 7 à 9, comprenant en outre des amorces et des sondes marquées pour détecter ou quantifier les acides nucléiques du VIH-1.

11. Utilisation du kit ou du mélange tel que défini dans les revendications 7 à 10, pour détecter ou quantifier les acides nucléiques du VIH-2 d'un échantillon biologique.

12. Procédé *in vitro* pour diagnostiquer une infection par le VIH-2, ou déterminer la charge virale du VIH-2, chez un individu, comprenant les étapes de :
(a) mettre en œuvre le procédé pour détecter ou quantifier les acides nucléiques du virus de l'immunodéficience humaine-2 (VIH-2) dans un échantillon biologique prélevé sur l'individu tel que défini dans les revendications 1 à 6 ;
(b) déterminer à partir de celui-ci si l'individu est infecté par le VIH-2 ou la charge virale de VIH-2 de l'individu.

13. Procédé *in vitro* pour déterminer si un individu est susceptible de bénéficier d'un traitement par thérapie antirétrovirale (ART) ou d'un ajustement d'ART, comprenant la mise en œuvre du procédé de diagnostic de l'infection par le VIH-2, ou de détermination de la charge virale de VIH-2, selon revendication 12.

14. Inhibiteurs nucléotidiques de transcriptase inverse (INTI), inhibiteurs de protéase (IP) et/ou inhibiteurs d'intégrase pour une utilisation dans la prévention ou le traitement de l'infection par le VIH-2 chez un individu, dans laquelle l'individu est déterminé comme étant susceptible de bénéficier d'un traitement par ART ou d'un ajustement d'ART par la mise en œuvre du procédé selon la revendication 13.

15. Sonde comprenant ou consistant en une séquence SEQ ID NO :3, ou une séquence complémentaire à la SEQ ID NO : 3.


### SEQUENCE LISTING

<110> UNIVERSITE PARIS DESCARTES
<120> IN VITRO METHOD FOR THE DETECTION AND QUANTIFICATION OF HIV-2
<130> B000011WO
<160> 9
<170> PatentIn version 3.5
<210> 1
   <211> 21
   <212> DNA
   <213> Artificial
<220>
   <223> PCR primer
<400> 1
   tctttaagca agcaagcgtg g 21
<210> 2
   <211> 21
   <212> DNA
   <213> Artificial
<220>
   <223> PCR primer
<400> 2
   agcaggtaga gcctgggtgt t 21
<210> 3
   <211> 20
   <212> DNA
   <213> Artificial
<220>
   <223> Probe
<400> 3
   cttggccggy rctgggcaga 20
<210> 4
   <211> 20
   <212> DNA
   <213> Artificial
<220>
   <223> PCR primer
<400> 4
   gcgcgagaaa ctccgtcttg 20
<210> 5
   <211> 22
   <212> DNA
   <213> Artificial
<220>
   <223> PCR primer
<400> 5
   ttcgctgccc acacaatatg tt 22
<210> 6
   <211> 23
   <212> DNA
   <213> Artificial
<220>
   <223> Probe
<400> 6
   taggttacgg cccggcggaa aga 23
<210> 7
   <211> 23
   <212> DNA
   <213> Artificial
<220>
   <223> PCR primer
<400> 7
   tccaacaggc tctctgctaa tcc 23
<210> 8
   <211> 297
   <212> DNA
   <213> Human immunodeficiency virus type 2
<400> 8
   cctcaattct ctctttggag gagaccagta gtcwcagcac acattgaggg ccagccagta    60
   gaagttttac tagatacagg ggccgacgac tcaatagtag caggggtaga gttaggaagc   120
   aattatagtc caaaaatagt agggggaata gggggattca taaataccaa agaatataag  180
   gatgtagaga taaaagtact aaataaaaca gtaagggcca ctataatgac aggtgaaacc  240
   ccaatcaaca tttttggcag aaacattttg acagcattag gcatgtcatt aaatcta      297
<210> 9
   <211> 23
   <212> DNA
   <213> Artificial
<220>
   <223> Probe
<400> 9
   tctttccgcc gggccgtaac cta 23